# EUROPEAN PATENT SPECIFICATION

(11) **EP 3 174 487 B1**
(45) Date of publication and mention of the grant of the patent: **08.07.2020**
(21) Application number: 15827739.2
(22) Date of filing: 26.06.2015
(51) Int. Cl.: A61B 18/18, A61N 5/02

(54) **SYSTEMS FOR IN SITU QUANTIFICATION OF A THERMAL ENVIRONMENT**
SYSTEME ZUR IN-SITU-QUANTIFIZIERUNG EINER THERMISCHEN UMGEBUNG
SYSTÈMES DE QUANTIFICATION IN SITU D'UN ENVIRONNEMENT THERMIQUE

(30) Priority: 31.07.2014 US 201462031230 P; 22.06.2015 US 201514745745
(43) Date of publication of application: 07.06.2017
(73) Proprietor: Covidien LP, Mansfield, MA 02048 (US)
(72) Inventor: LADTKOW, Casey M., Erie, CO 80516 (US); BRANNAN, Joseph D., Erie, CO 80516 (US); DICKHANS, William J., Longmont, CO 80503 (US)
(74) Representative: Maschio, Antonio
(86) International application number: PCT/US2015/037891
(87) International publication number: WO 2016/018546

(56) References cited:
- US-A1- 2001 016 762
- US-A1- 2008 139 931
- US-A1- 2009 221 932
- US-A1- 2010 185 191
- US-A1- 2012 035 603
- US-A1- 2012 179 048
- US-A1- 2013 204 240
- US-A1- 2013 272 339
- US-A1- 2013 272 339
- US-A1- 2013 345 693
- US-B2- 8 447 385

## Description

### BACKGROUND

### 1. Technical Field

The present disclosure relates to microwave thermometry in microwave ablation systems and, more particularly, to systems and methods for in-situ quantification of a thermal environment surrounding tissue.

### 2. Discussion of Related Art

Electromagnetic radiation can be used to heat and destroy tumor cells. Treatment may involve inserting ablation probes into tissues where cancerous tumors have been identified. Once the ablation probes are properly positioned, the ablation probes emit electromagnetic radiation into tissue surrounding the ablation probes.

In the treatment of diseases such as cancer, certain types of tumor cells have been found to denature at elevated temperatures that are slightly lower than temperatures normally injurious to healthy cells.

Electrosurgical devices utilizing electromagnetic radiation have been developed for a variety of uses and applications. Typically, apparatus for use in ablation procedures include a power generation source, e.g., a microwave or radio frequency (RF) electrosurgical generator that functions as an energy source and a surgical instrument (e.g., microwave ablation probe having an antenna assembly) for directing energy to the target tissue. The generator and surgical instrument are typically operatively coupled by a cable assembly having a plurality of conductors for transmitting energy from the generator to the instrument, and for communicating control, feedback and identification signals between the instrument and the generator.

There are several types of microwave probes in use, e.g., monopole, dipole, and helical, which may be used in tissue ablation applications. In monopole and dipole antenna assemblies, microwave energy generally radiates perpendicularly away from the axis of the conductor. Monopole antenna assemblies typically include a single, elongated conductor. A typical dipole antenna assembly includes two elongated conductors that are linearly-aligned and positioned end-to-end relative to one another with an electrical insulator placed therebetween. Helical antenna assemblies include helically-shaped conductor configurations of various dimensions, e.g., diameter and length. The main modes of operation of a helical antenna assembly are normal mode (broadside), in which the field radiated by the helix is maximum in a perpendicular plane to the helix axis, and axial mode (end fire), in which maximum radiation is along the helix axis.

The particular type of tissue ablation procedure may dictate a particular ablation volume in order to achieve a desired surgical outcome. Ablation volume is correlated with antenna design, antenna performance, antenna impedance, ablation time and wattage, and tissue characteristics, e.g., tissue impedance. Any ablation volume is characterized by having a number of different temperature ranges. Measurement of the different temperature ranges would provide valuable information to the surgeon.

Accordingly, there is a need for a device for use during a surgical procedure that will measure and indicate the thermal fields around tissue. In this context, reference is made to document US2013/0272339 A1.

### SUMMARY

The invention is defined in the appended set of claims. In an aspect of the present disclosure, a microwave ablation system is presented including a microwave applicator including an antenna configured to deliver microwave energy, a microwave generator coupled to the microwave applicator and configured to generate a microwave signal and transmit the microwave signal to the antenna, and a radiometer configured to measure emissions from a thermal field created when the microwave applicator delivers microwave energy, the thermal field providing in-situ quantitative information of a material in the thermal field.

In some aspects, the radiometer is incorporated within the microwave applicator. Alternatively, the radiometer is separate and distinct from the microwave applicator.

In certain aspects, the antenna of the microwave applicator includes a single temperature sensor. Alternatively, the antenna of the microwave applicator includes an array of temperature sensors.

In aspects, the thermal field is displayed on a display monitor.

In some aspects, the quantitative information is at least one of thermal conductivity, specific heat, density, and blood perfusion rate.

In another aspect of the present disclosure, the quantitative information is used to automatically adjust power transmission and time settings of the microwave generator to compensate for different thermal environments.

In some aspects, the thermal environment dictates a response to the energy applied by the microwave applicator.

In still other aspects of the present disclosure, a method for assessing a thermal environment is presented, the method including the steps of radiating energy from a microwave applicator for a predetermined amount of time, measuring a thermal field via a radiometer, and obtaining in-situ quantitative information of the thermal environment.

Further scope of applicability of the present disclosure will become apparent from the detailed description given hereinafter. However, it should be understood that the detailed description and specific examples, while indicating illustrative embodiments of the present disclosure, are given by way of illustration only, since various changes and modifications within the scope of the present disclosure will become apparent to those skilled in the art from this detailed description.

### BRIEF DESCRIPTION OF THE DRAWINGS

Various aspects of the present disclosure are described hereinbelow with reference to the drawings, wherein:
FIG. 1 is a block diagram of a microwave ablation system, in accordance with embodiments of the present disclosure;
FIG. 2 depicts a microwave generator and a microwave applicator that incorporates the radiometer of the microwave ablation system of FIG. 1, in accordance with an embodiment of the present disclosure;
FIG. 3 depicts a microwave generator and a microwave applicator, where a radiometer is separate from the microwave applicator, in accordance with an embodiment of the present disclosure;
FIG. 4 depicts the microwave applicator of FIG. 2 approaching a material, such as tissue having different thermal fields, in accordance with an embodiment of the present disclosure;
FIG. 5 depicts the distal end of the microwave applicator of FIG. 2 penetrating tissue, in accordance with an embodiment of the present disclosure;
FIG. 6 depicts a display for displaying the different thermal fields of the tissue of FIG. 4, in accordance with an embodiment of the present disclosure;
FIG. 7 depicts the distal end of the microwave applicator of FIG. 2 penetrating the tissue of FIG. 5, where energy bands are shown around the distal end of the microwave applicator, in accordance with an embodiment of the present disclosure;
FIG. 8 depicts a perspective view of a microwave applicator including a radiometer controller, in accordance with an embodiment of the present disclosure;
FIG. 9 depicts a block diagram of a microwave ablation system in accordance with other embodiments of the present disclosure; and
FIG. 10 illustrates a flowchart of a method of measuring the thermal field using radiometer measurements, in accordance with an embodiment of the present disclosure.

The figures depict illustrative embodiments of the present disclosure for purposes of illustration only. One skilled in the art will readily recognize from the following detailed description that alternative embodiments of the structures and methods illustrated herein may be employed without departing from the principles of the present disclosure.

### DETAILED DESCRIPTION

The present disclosure is generally directed to microwave ablation systems that incorporate microwave radiometry systems for the physiological environment and for identifying a thermal field surrounding a microwave applicator. Microwave radiometry is a technique for measuring electromagnetic energy considered as thermal radiation, and can be used to detect and measure microwave energy emanating from heat sources.

The microwave ablation systems according to the present disclosure allow for a thermal environment to be created related to target tissue. The thermal environment may be assessed, in real-time, to allow the clinician to instantaneously receive feedback, and use that feedback to evaluate different approaches to use during the surgical procedure. Therefore, application of thermal therapy can be continuously adjusted or modified or altered based on real-time feedback related to the thermal zones of tissue that form the thermal environment. As such, the thermal environment dictates energy response applied by the surgical instrument (e.g., microwave applicator).

Embodiments of the microwave ablation systems and components are described with reference to the accompanying drawings. Like reference numerals may refer to similar or identical elements throughout the description of the figures. As shown in the drawings and as used in this description, the term "proximal" refers to that portion of the apparatus, or component of the apparatus, closer to the user and the term "distal" refers to that portion of the apparatus, or component thereof, farther from the user.

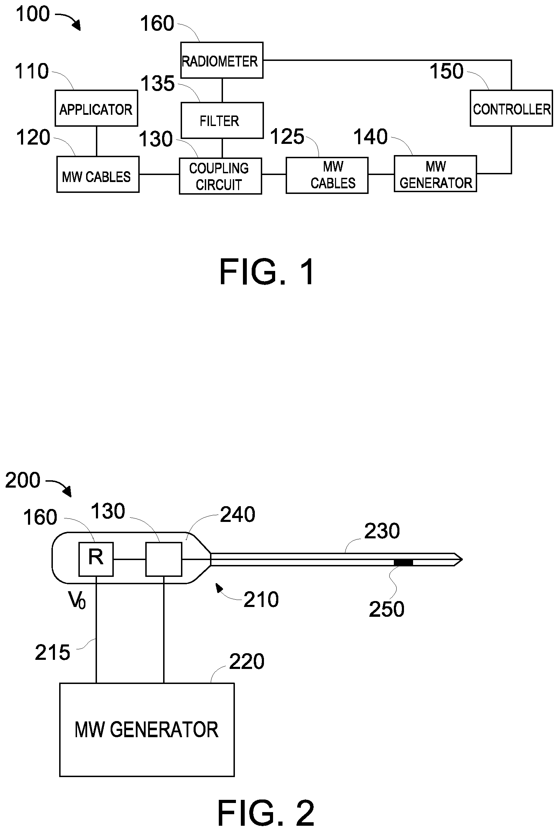

FIG. 1 is a block diagram of a microwave ablation system 100 according to embodiments of the present disclosure. The microwave ablation system 100 includes a microwave applicator 110, microwave cables 120 and 125, a coupling circuit 130, a microwave generator 140, a filter 135, and a radiometer 160. The microwave generator 140 generates a microwave signal and outputs it to the microwave applicator 110 via the microwave cables 120 and 125. The microwave applicator 110 includes at least one antenna which emits microwave radiation when the microwave signal is applied to the antenna. The antenna may be disposed in a tumor so that the microwave radiation emitted from the antenna can ablate the tumor.

The microwave applicator 110, as is common for microwave antennas, acts as both a transmitter and a receiver. All materials give off some radiation. When that material such as tissue is heated, the change in temperature results in an increase in the intensity or frequency of those emissions. These emissions are one type of radiation that can be received by a microwave antenna, such as microwave applicator 110. Generally, these received emissions are considered noise or noise temperature signals in the system in which they are received, and, indeed, the components of the system including the microwave applicator 110 and microwave cables 120, 125 themselves give off an emission of noise as they are used. These emissions, however, can provide indications of quantitative information of the material in question. The quantitative information may relate to at least thermal conductivity, specific heat, density, and local perfusion of tissue. The quantitative information may be used to automatically adjust power transmission and time settings of the microwave generator to compensate for different thermal environments. Moreover, the thermal environment can dictate the tissue's response to the energy applied by the microwave applicator 110.

Regarding the quantitative information, the thermal conductivity of biological tissue is dependent on the particular type of biological tissue and on the composition of the biological tissue. Different biological tissues exhibit different and/or unique thermal conductivity based on factors such as tissue density, vascularization, age, direction and distance to major blood vessels, etc. Additionally, different biological tissues may exhibit a different and/or unique thermal conductivity in different directions. Electrical conductivity is not only determined by tissue type and composition, but also by other externally applied physical and chemical influences during thermal treatment, such as, for example, temperature inducement and saline pretreatment. Knowing such characteristics of tissue allow for the creation of and quantification of one or more thermal fields in tissue following the application of energy to the tissue.

Therefore, thermal zones based on these emissions of the tissue following application of energy may be quantified and displayed on a display screen, thus allowing a surgeon to create and visualize the thermal environment surrounding a microwave applicator during a surgical procedure. Stated differently, the thermal environment may be assessed, in real-time, to allow the surgeon to instantaneously receive feedback, and use that feedback to evaluate different approaches to use during the surgical procedure. Therefore, application of thermal therapy can be continuously adjusted or modified or altered based on real-time feedback related to the thermal zones of tissue that form a thermal environment. As such, the thermal environment dictates energy response applied by the surgical instrument (e.g., microwave applicator 110).

Moreover, multiple thermal environments may be simultaneously assessed during a single surgical procedure. In other words, a surgeon may assess a thermal environment of a plurality of different tissues or organs and compare such thermal environments to determine appropriate action during the surgical procedure. Therefore, the real-time assessment of a plurality of thermal environments influences a surgeon's decision on, for example, how much energy to apply to each of the plurality of tissues. In other words, a direct relationship is established between one or more computed thermal environments of tissue and subsequent energy application to such tissue. Stated differently, thermal characteristics of tissue are used to create one or more thermal fields that form a thermal environment that is displayed to allow for real-time, instantaneous, and continuous adjustments to be made to the computed thermal environments. Such adjustments may relate to, for instance, power adjustments or time-setting adjustments. Of course, one skilled in the art may contemplate making any type of variable or parameter adjustments, continuously and in real-time, to the computed thermal fields.

The coupling circuit 130 is coupled between the microwave generator 140 and the microwave applicator 110 and receives noise temperature signals or at least a portion of the signals propagating through the microwave cables 120 and 125, and directs the noise temperature signals to the radiometer 160, including those which are received by the microwave applicator 110 from tissue being treated, such as a tumor. The filter 135 isolates the noise temperature signals from the microwave signal generated by the microwave generator 150, including any reflected power resulting from an imbalance between the source and the load impedance. Then, the radiometer 160 samples the noise temperature signals and provides them to the controller 150. The controller 150 may convert the microwave noise temperature signal into a temperature reading by digitally sampling the microwave noise temperature signal using an analog-to-digital converter (ADC) and scaling the result. The controller 150 may also interface with a display to display the temperature reading as described in more detail below.

The noise temperature measured by the radiometer 160 may be used to enable temperature feedback control. The feedback control may involve open loop control, e.g., user-based control, or closed loop control, e.g., for an autonomous system, to achieve a desired tissue effect and to improve the overall procedural outcome. The radiometer 160 and controller 150 may also be used to monitor the temperature of components of the microwave ablation system 100. For example, the radiometer 160 and controller 150 may be used to monitor the temperature of the microwave cables 120, 125 to insure adequate cooling and to avoid failures. Further, in accordance with at least one embodiment of the present disclosure, the noise temperatures of components and tissue are identified, separated from one another, and used to control aspects of the microwave ablation system 100.

FIG. 2 is a block diagram of a microwave ablation system 200 according to some embodiments of the present disclosure. The microwave ablation system 200 includes a microwave generator 220 and a microwave applicator 210 coupled to the microwave generator 220. The microwave applicator 210 includes a microwave antenna 230 and a handle 240 coupled to the microwave antenna 230 to allow a clinician to manipulate the microwave antenna 230 during a microwave ablation procedure.

The microwave antenna 230 may be embodied as an inflexible ablation catheter or a flexible ablation catheter to accommodate a specific surgical procedure, a specific luminal structure, specific target tissue, a clinician's preference, etc. For example, in one embodiment, it may prove advantageous to have an ablation catheter that is very flexible for movement through the relatively narrow airways of the lungs of a patient. In some cases, it may prove advantageous to have an ablation catheter that is only slightly flexible, e.g., where the ablation catheter is needed to pierce or puncture tissue. It should be understood to those of skill in the art that the microwave antenna 230 may employ other ablation catheter embodiments, either simplified or more complex in structural detail, without departing from the scope of the instant disclosure.

To obtain accurate temperature measurements, the radiometer 160 is disposed as close as possible to the radiating portion of the microwave antenna 230 to limit unwanted noise (e.g., from heating of a coaxial cable employed in most microwave antennas) from entering the radiometer 160. For example, as shown in FIG. 2, the radiometer 160 and the coupling circuit 130 are disposed within the handle 240 of the microwave applicator 210. The coupling circuit 130 is coupled between microwave feed transmission line and the antenna element to provide at least a portion of a microwave signal propagating in the antenna element to the radiometer 160. The radiometer 160 is coupled to the coupling circuit 130. The radiometer may output a voltage signal Vo or a digital signal derived from the temperature of the environment surrounding the antenna 230, e.g., tissue that is being ablated. That is, the environment having been heated by the microwave antenna emits a noise temperature signal at a particular frequency, and the temperature of the environment may be derived from the intensity of that signal. This voltage signal V₀ or digital signal is provided to the microwave generator 220 via communication line 215.

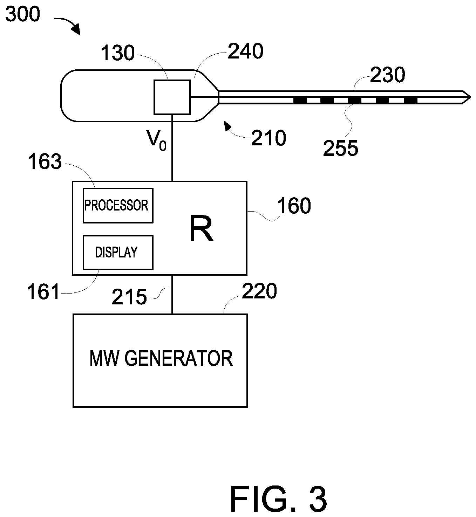

FIG. 3 depicts a microwave generator and a microwave applicator, where the radiometer is separate from the microwave applicator, in accordance with an embodiment of the present disclosure. The microwave ablation system 300 includes a microwave generator 220 and a microwave applicator 210 coupled to the microwave generator 220. The microwave applicator 210 includes a microwave antenna 230 and a handle 240 coupled to the microwave antenna 230 to allow a clinician to manipulate the microwave antenna 230 during a microwave ablation procedure.

To obtain accurate temperature measurements, the radiometer 160 is disposed exterior to the microwave ablation system 300. Effectively, this isolates the radiometer from at least some of the noise of the microwave ablation system 300. For example, as shown in FIG. 3, the radiometer 160 is disposed between the microwave applicator 210 and the microwave generator 220. The radiometer 160 may include a display means 161 and a processor (or controller) 163 therein. Therefore, in one exemplary embodiment, the radiometer 160 may be separate and distinct from the microwave applicator 210.

Moreover, regarding FIGS. 2 and 3, the antenna 230 of the microwave applicator 210 may include one or more temperature sensors. For example, the antenna of FIG. 2 may include a single temperature sensor 250, whereas the antenna of FIG. 3 may include an array of temperature sensors 255, the temperature sensors 250, 255 may be thermocouples and connect to the controller to provide additional temperature data of the antenna 230, cooling channels (not shown) within the cooling channel, or of specific components, such as a balun or choke formed within the antenna 230. Of course, any of the microwave applicators of the present disclosure may include any number of temperature sensors incorporated on any portion of the microwave applicator 210.

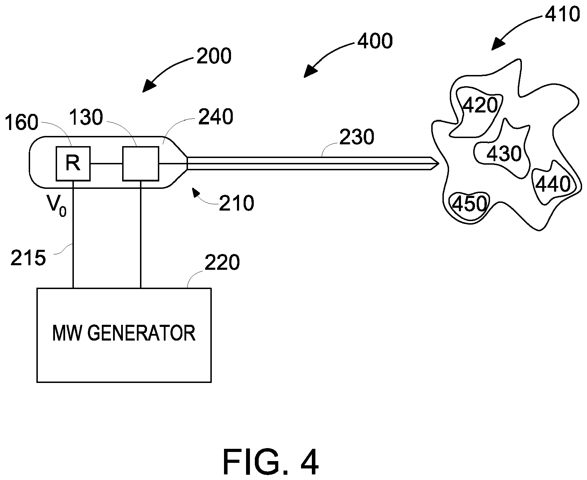

FIG. 4 depicts the microwave applicator 210 of FIG. 2 approaching tissue 410 having different thermal fields, in accordance with an embodiment of the present disclosure. The tissue 410 may include a plurality of thermal areas or regions or zones 420, 430, 440, 450. Each thermal zone 420, 430, 440, 450 may exhibit different tissue characteristics. For example, each thermal zone 420, 430, 440, 450 may have a different temperature or temperature range. One common reason for different thermal zones within tissue is that the tissue in question is of a different type or has a different composition. For example, cancerous lesions and tumors typically have heightened vasculature as compared to healthy tissue. As a result, lesions and tumors typically have a greater water content and blood content than surrounding tissue resulting in different emissions than healthy tissue. Similarly, bone has a very different emissions signature than muscle tissue, and these distinctions can be discerned by analyzing the emissions using radiometer 160. Of course, the tissue characteristics are not limited only to these examples. The tissue characteristics may also include, for instance, tissue volume measurements and tissue density measurements.

The differences in emissions may be useful in guiding the microwave applicator 210 to, for example, place the microwave applicator 210 within tissue expressing a particular emission representative of particular tissue characteristics. In addition, certain types of tissue may be avoided when placing the microwave applicator 210.

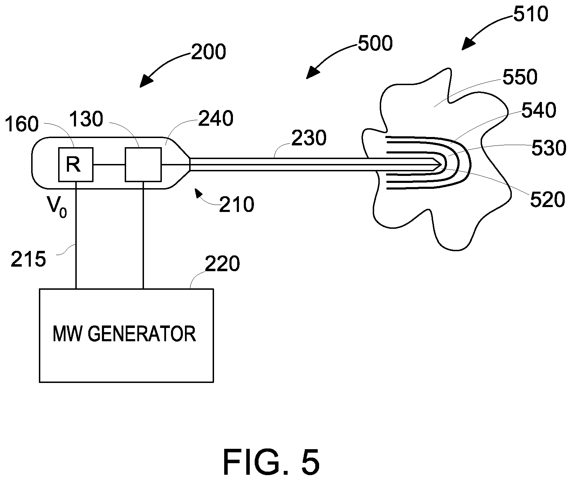

FIG. 5 depicts the distal end of the microwave applicator of FIG. 2 penetrating and applying energy to tissue, in accordance with an embodiment of the present disclosure.

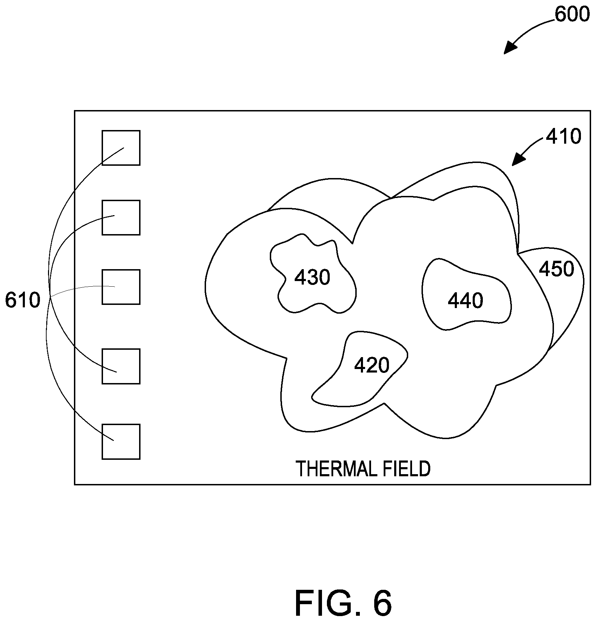

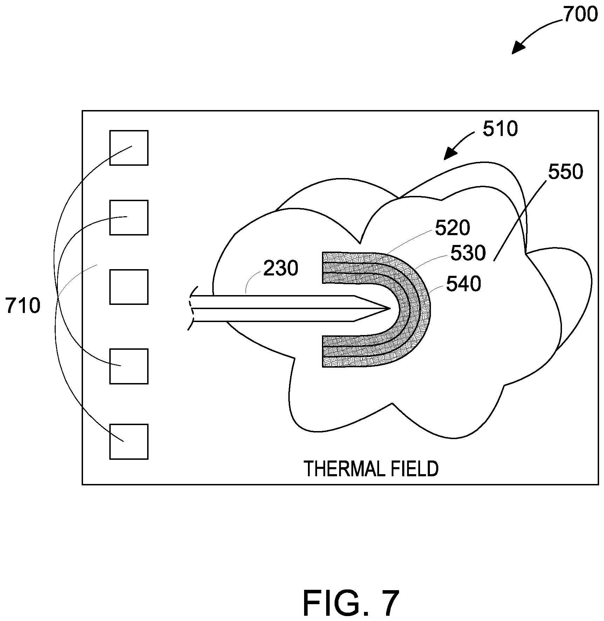

In the system 500, the distal end of the microwave applicator 210 penetrates the tissue 510 and approaches, for example, a thermal zone 550, the emissions of which may be sensed based before entry and application of energy or as a result of an interrogating signal from the microwave applicator 210 to which a response is received from the tissue identifying thermal zone 550. When the distal end is in the vicinity of, adjacent to, or inserted into the thermal zone 550 (i.e., a particular type of tissue such as a tumor), energy is applied to the tissue resulting in the creation of energy bands 520, 530, 540. Energy bands 520, 530, and 540 are representative of the temperature achieved by the application of energy. Each energy band 520, 530, 540 may be determined by looking for emissions over time (i.e., greater time means the sensed emissions come from tissue further from microwave applicator 210). Alternatively, each energy band 520, 530, 540 may be emitting at a different frequency or a different intensity and from these measurements looking at variations over time, in frequency, or in intensity, determinations can be made as to the temperature of the tissue. The energy bands 520, 530, 540 may be displayed on a display monitor (see FIGS. 6 and 7). The energy bands 520, 530, 540 are indicative of different thermal characteristics of the thermal zone 550 and may be represented as different colors. As discussed below, the energy bands 520, 530, 540 form a thermal field related to the thermal zone 550 approached by the microwave applicator 210. The thermal field is a representation of a thermal environment surrounding the tissue.

The radiometric emissions, as described above, may be fed back to the controller 150 (FIG. 1) to control microwave generator 140. However, this emissions data may also be manipulated by the controller 150 to enhance a display of the procedure as depicted in FIG. 6.

The display 600 illustrates a plurality of input means 610 for manipulating the three-dimensional view of the tissue 410. The tissue 410 includes, for example, a plurality of thermal zones 420, 430, 440, 450 that form different thermal fields within the tissue 410. The image of the tissue 410 may be provided from prior imaging modalities such as computed tomography (CT) scan, magnetic resonance imaging, fluoroscopy, ultrasound imaging, and others. As shown, the display screen 600 illustrates a 3D view of the tissue 410 having the microwave applicator 210 (as seen in FIG. 2) inserted. More details on systems and methods for navigating and placement of a microwave applicator at a location for treatment may be found in co-pending U.S. Application Serial No. XX/XXX,XXX (attorney docket no. H-IL-00137 (1988-137)) entitled "System and Method for Navigating within the Lung," filed concurrently with the present application, and U.S. Patent Application 13/838,805, filed on March 15, 2013, entitled "Pathway Planning System and Method," the entire contents of which are incorporated herein by reference. The input means 610 may permit a user to, for example, rotate the tissue, zoom in and out of specific thermal zones, measure the thermal fields, count the thermal fields, extract further tissue information regarding each thermal zone, etc.

A user, such as a surgeon, can identify, view, and access each thermal zone, and extract any desirable tissue characteristics from each selected thermal zone. Thus, quantitative information may be separately extracted for each thermal zone. The quantitative information extracted from each thermal zone may be collected and stored in a memory device. The quantitative information of each thermal zone may be compared to tissue characteristics of healthy tissue. Also, the quantitative information of each thermal zone may be compared to each other. Therefore, a surgeon may, instantaneously and in real-time, compare different tissue areas/regions or thermal zones of a tissue, and extract valuable quantitative information regarding the thermal environment. This information regarding the thermal zones may be particularly useful for setting parameters of a microwave generator 220 or setting a treatment program for the application of microwave energy to treat a target located in one or more of these thermal zones.

FIG. 7 depicts the distal end of the microwave applicator of FIG. 2 penetrating the tissue of FIG. 5, following the application of energy from the microwave generator 220 where energy bands are shown around the distal end of the microwave antenna 230, in accordance with an embodiment of the present disclosure.

The display 700 illustrates a plurality of input means 710 that are used to identify and quantify the temperature of a thermal zone 550 of tissue (see FIG. 5). When the distal end of the microwave applicator 210 (i.e., antenna 230) penetrates the tissue 510 and applies energy to thermal zone 550, energy bands 520, 530, 540 are created representing the change in temperature of those portions of the thermal zone 550 and tissue 510. These thermal bands 520, 530, and 540 may be displayed on the display screen 700 around the distal end of the microwave antenna 230. The energy bands 520, 530, 540 form a thermal field providing in-situ quantitative information of the thermal environment of the tissue 510.

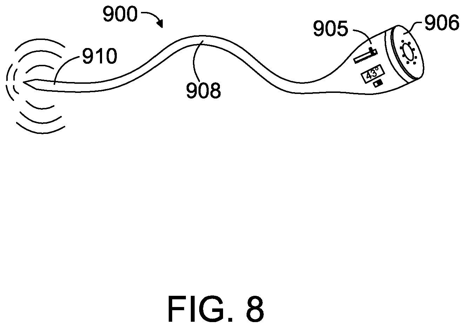

FIG. 8 depicts a perspective view of a microwave applicator including a radiometer controller, in accordance with an embodiment of the present disclosure.

The microwave applicator 900 incorporates a microwave radiometry module into the microwave applicator's connector assembly 905. The connector assembly 905 is connected to a probe 908 having a radiating portion 910. The connector assembly 905 includes a display that displays temperature measurements and a user interface that allows a user to change temperature settings and to shutoff microwave signals being provided to the probe 908 to cause the radiating portion 910 to emit microwave radiation. The connector assembly 905 includes a connector 906 that is configured to connect directly to a microwave generator or to connect to the microwave generator via a microwave cable. Radiometric data such as temperature may be transmitted to the microwave generator via the data bus of the connector 906.

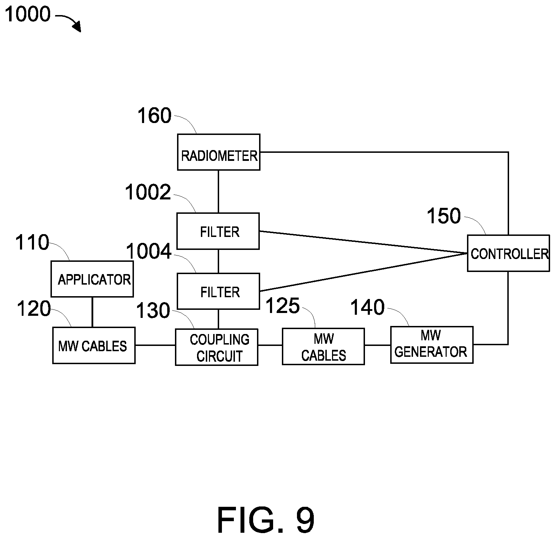

FIG. 9 depicts a block diagram of a microwave ablation system in accordance with other embodiments of the present disclosure.

The microwave ablation system 1000 is the same as the microwave ablation system 100 shown in FIG. 1 except that the microwave ablation system 1000 includes filters 1002 and 1004, which are controlled by the controller 150. The first filter 1002 may separate the noise temperature signal from the high power microwave signal. The second filter 1004 may then extract the noise temperature of the tissue and the noise temperature of the transmission network from the noise temperature output from the first filter 1002. One or more additional filters may be employed without departing from the scope of the present disclosure.

The controller 150 may provide tuning, gating, and other signals to control the manner in which the first filter 1002 and the second filter 1004 filter the microwave energy received by the coupling circuit. The second filter 1004 may be further configured to separate out components of the transmission network noise temperature or the tissue noise temperature. For example, different components of the transmission network may produce noise temperature signals at different frequencies. The second filter 1004 may employ frequency domain techniques to determine the noise temperature of each of the components of the transmission network by analyzing the noise temperature signals at different frequencies. The second filter 1004 may alternatively employ both time domain and frequency domain techniques to isolate noise temperature signals from intentional sources from other noise temperature sources, such as the cables and the tissue.

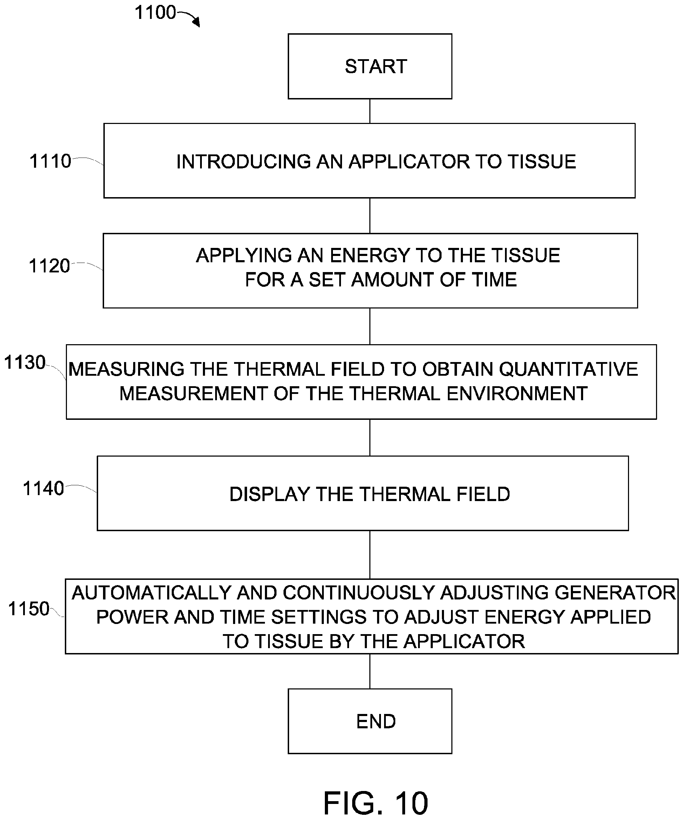

FIG. 10 illustrates a flowchart 1100 of a method of measuring the thermal field using radiometer measurements, in accordance with an embodiment of the present disclosure.

In step 1110, a microwave applicator is introduced in tissue. In step 1120, microwave energy is applied to the tissue for a set amount of time. In step 1130, the thermal field is measured to obtain a quantitative measurement of the thermal environment. In step 1140, the thermal field is displayed on a display screen. In step 1150, generator power and time settings are automatically and continuously adjusted in order to adjust the energy applied to the tissue by the microwave applicator. The process then ends.

It is to be understood that the method steps described herein need not necessarily be performed in the order as described. Further, words such as "thereafter," "then," "next," etc. are not intended to limit the order of the steps. These words are simply used to guide the reader through the description of the method steps.

Further, the features and aspects of the present disclosure may be implemented in microwave ablation system 100 in any suitable fashion, e.g., via the hardware and software configuration of microwave ablation system 100 or using any other suitable software, firmware, and/or hardware.

For instance, when implemented via executable instructions, various elements of the present disclosure are in essence the code defining the operations of such various elements. The executable instructions or code may be obtained from a readable medium (e.g., a hard drive media, optical media, EPROM, EEPROM, tape media, cartridge media, flash memory, ROM, memory stick, and/or the like) or communicated via a data signal from a communication medium (e.g., the Internet). In fact, readable media may include any medium that may store or transfer information.

The computer means or computing means or processing means may be operatively associated with the assembly, and is directed by software to compare the first output signal with a first control image and the second output signal with a second control image. The software further directs the computer to produce diagnostic output. Further, a means for transmitting the diagnostic output to an operator of the verification device is included. Thus, many applications of the present disclosure could be formulated. The exemplary network disclosed herein may include any system for exchanging data or transacting business, such as the Internet, an intranet, an extranet, WAN (wide area network), LAN (local area network), satellite communications, and/or the like. It is noted that the network may be implemented as other types of networks.

Additionally, "code" as used herein, or "program" as used herein, may be any plurality of binary values or any executable, interpreted or compiled code which may be used by a computer or execution device to perform a task. This code or program may be written in any one of several known computer languages. A "computer," as used herein, may mean any device which stores, processes, routes, manipulates, or performs like operation on data. A "computer" may be incorporated within one or more transponder recognition and collection systems or servers to operate one or more processors to run the transponder recognition algorithms. Moreover, computer-executable instructions include, for example, instructions and data which cause a general purpose computer, special purpose computer, or special purpose processing device to perform a certain function or group of functions. Computer-executable instructions also include program modules that may be executed by computers in stand-alone or network environments. Generally, program modules include routines, programs, objects, components, and data structures, etc. that perform particular tasks or implement particular abstract data types.

This description uses the phrases "in an embodiment," "in embodiments," "in some embodiments," or "in other embodiments," which may each refer to one or more of the same or different embodiments in accordance with the present disclosure.

Electromagnetic energy is generally classified by increasing energy or decreasing wavelength into radio waves, microwaves, infrared, visible light, ultraviolet, X-rays and gamma-rays. As it is used in this description, "microwave" generally refers to electromagnetic waves in the frequency range of 300 megahertz (MHz) (3×0⁸ cycles/second) to 300 gigahertz (GHz) (3×10¹¹ cycles/second). As it is used in this description, "ablation procedure" generally refers to any ablation procedure, such as, for example, microwave ablation, radiofrequency (RF) ablation, or microwave or RF ablation-assisted resection.

As it is used in this description, "energy applicator" generally refers to any device that can be used to transfer energy from a power generating source, such as a microwave or RF electrosurgical generator, to tissue. For the purposes of the present disclosure, the term "energy applicator" is interchangeable with the term "energy-delivery device." As it is used in this description, "transmission line" generally refers to any transmission medium that can be used for the propagation of signals from one point to another. As it is used in this description, "fluid" generally refers to a liquid, a gas or both.

As it is used in this description, the term "controller" refers to any electrical device that employs digital and/or analog components to generate digital and/or analog signals to control or drive another device. The term "controller" may refer to a digital signal processor, a microcontroller, or a computer having a processor, a memory, and input/output ports for carrying out some of the methods described herein.

The word "exemplary" is used herein to mean "serving as an example, instance, or illustration." Any embodiment described herein as "exemplary" is not necessarily to be construed as preferred or advantageous over other embodiments. The word "example" may be used interchangeably with the term "exemplary."

Persons skilled in the art will understand that the devices and methods specifically described herein and illustrated in the accompanying drawings are nonlimiting exemplary embodiments. The features illustrated or described in connection with one exemplary embodiment may be combined with the features of other embodiments. Such modifications and variations are intended to be included within the scope of the present disclosure.

The foregoing examples illustrate various aspects of the present disclosure and practice of the methods of the present disclosure. The examples are not intended to provide an exhaustive description of the many different embodiments of the present disclosure. Thus, although the foregoing present disclosure has been described in some detail by way of illustration and example for purposes of clarity and understanding, those of ordinary skill in the art will realize readily that many changes and modifications may be made thereto without departing form the scope of the present disclosure.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

## Claims

1. A microwave ablation system, comprising:
a display;
a microwave applicator (110) including an antenna configured to deliver microwave energy;
a microwave generator (140) coupled to the microwave applicator and configured to generate a microwave signal and transmit the microwave signal to the antenna; and
a radiometer (160) configured to measure emissions from a plurality of thermal bands (520, 530, 540) created around a distal end of the microwave applicator when the microwave applicator delivers microwave energy, the plurality of thermal bands forming a thermal field, being represented on the display with different colours, and providing in-situ quantitative information of a material in the thermal field;
wherein the radiometer is (i) incorporated within the microwave applicator, or (ii) disposed between the microwave applicator and the microwave generator.

2. The microwave ablation system according to claim 1, wherein the radiometer is incorporated within the microwave applicator.

3. The microwave ablation system according to claim 2, wherein the radiometer is disposed in a handle (240) of the microwave applicator.

4. The microwave ablation system according to claim 2, wherein the radiometer is incorporated into the connector assembly of the microwave applicator.

5. The microwave ablation system according to any preceding claim, wherein the antenna (230) of the microwave applicator includes a single temperature sensor (250).

6. The microwave ablation system according to any of claims 1 to 4, wherein the antenna of the microwave applicator includes an array of temperature sensors (255).

7. The microwave ablation system according to any preceding claim, wherein the quantitative information is at least one of thermal conductivity, specific heat, density, and blood perfusion rate.

8. The microwave ablation system according to any preceding claim, wherein the quantitative information is used to automatically adjust power transmission and time settings of the microwave generator to compensate for different thermal environments.

## Patentansprüche

1. Mikrowellenablationssystem, umfassend:
ein Anzeigegerät;
einen Mikrowellenapplikator (110) mit einer Antenne, der dafür eingerichtet ist, Mikrowellenenergie abzugeben,
einen Mikrowellengenerator (140), der mit dem Mikrowellenapplikator gekoppelt und dafür eingerichtet ist, ein Mikrowellensignal zu erzeugen und das Mikrowellensignal zu der Antenne zu übertragen; und
einen Strahlungsmesser (160), der dafür eingerichtet ist, Abstrahlungen aus einer Vielzahl von thermischen Bändern (520, 530, 540) zu messen, die um ein distales Ende des Mikrowellenapplikators herum erzeugt werden, wenn der Mikrowellenapplikator Mikrowellenenergie abgibt, wobei die Vielzahl von thermischen Bändern ein thermisches Feld bildet, das auf dem Anzeigegerät mit verschiedenen Farben dargestellt wird und *in situ* quantitative Informationen über ein Material in dem thermischen Feld liefert;
wobei der Strahlungsmesser (i) in den Mikrowellenapplikator eingebaut ist oder (ii) zwischen dem Mikrowellenapplikator und dem Mikrowellengenerator angeordnet ist.

2. Mikrowellenablationssystem nach Anspruch 1, wobei der Strahlungsmesser in den Mikrowellenapplikator eingebaut ist.

3. Mikrowellenablationssystem nach Anspruch 2, wobei der Strahlungsmesser in einem Griff (240) des Mikrowellenapplikators angeordnet ist.

4. Mikrowellenablationssystem nach Anspruch 2, wobei der Strahlungsmesser in die Anschlussstecker-Baugruppe des Mikrowellenapplikators eingebaut ist.

5. Mikrowellenablationssystem nach einem der vorstehenden Ansprüche, wobei die Antenne (230) des Mikrowellenapplikators einen einzigen Temperatursensor (250) einschließt.

6. Mikrowellenablationssystem nach einem der Ansprüche 1 bis 4, wobei die Antenne des Mikrowellenapplikators ein Array von Temperatursensoren (255) einschließt.

7. Mikrowellenablationssystem nach einem der vorstehenden Ansprüche, wobei es sich bei den quantitativen Informationen um mindestens eine von Wärmeleitfähigkeit, spezifischer Wärme, Dichte und Blutperfusionsrate handelt.

8. Mikrowellenablationssystem nach einem der vorstehenden Ansprüche, wobei die quantitativen Informationen zum selbsttätigen Einstellen von Energieübertragung und Zeiteinstellungen des Mikrowellengenerators verwendet werden, um unterschiedliche thermische Umgebungen auszugleichen.

## Revendications

1. Système d'ablation par microondes comprenant :
un écran ;
un applicateur de microondes (110) comportant une antenne configurée pour délivrer l'énergie à microondes ;
un générateur de microondes (140) couplé à l'applicateur de microondes et configuré pour générer un signal de microondes et transmettre le signal de microondes à l'antenne ; et
un radiomètre (160) configuré pour mesurer les émissions à partir d'une pluralité de bandes thermiques (520, 530, 540) créées autour d'une extrémité distale de l'applicateur de microondes lorsque celui-ci délivre de l'énergie à microondes, la pluralité de bandes thermiques formant un champ thermique, étant représentée sur l'écran avec différentes couleurs et fournissant des informations quantitatives in situ d'un matériau dans le champ thermique ;
dans lequel le radiomètre est (i) incorporé à l'intérieur de l'applicateur de microondes, ou (ii) disposé entre l'applicateur de microondes et le générateur de microondes.

2. Système d'ablation par microondes selon la revendication 1, dans lequel le radiomètre est incorporé à l'intérieur de l'applicateur de microondes.

3. Système d'ablation par microondes selon la revendication 2, dans lequel le radiomètre est disposé dans une poignée (240) de l'applicateur de microondes.

4. Système d'ablation par microondes selon la revendication 2, dans lequel le radiomètre est incorporé dans l'ensemble connecteur de l'applicateur de microondes.

5. Système d'ablation par microondes selon l'une quelconque des revendications précédentes, dans lequel l'antenne (230) de l'applicateur de microondes comprend un seul capteur de température (250).

6. Système d'ablation par microondes selon l'une des revendications 1 à 4, dans lequel l'antenne de l'applicateur de microondes comprend un réseau de capteurs de température (255).

7. Système d'ablation par microondes selon une quelconque revendication précédente, dans lequel les informations quantitatives sont au moins une parmi la conductivité thermique, la chaleur spécifique, la densité et le taux de perfusion sanguine.

8. Système d'ablation par microondes selon une quelconque revendication précédente, dans lequel les informations quantitatives sont utilisées pour ajuster automatiquement les réglages de transmission de puissance et de temps du générateur de microondes afin de compenser les différents environnements thermiques.
